Europäisches Patentamt

European Patent Office

Office européen des brevets

(19)

(11) Veröffentlichungsnummer : **0 379 949 B1**

# (12) EUROPÄISCHE PATENTSCHRIFT

(45) Veröffentlichungstag der Patentschrift :
**29.03.95 Patentblatt 95/13**

(51) Int. Cl.$^6$ : **G01N 33/569,** G01N 33/543,
G01N 33/563, C07K 7/00

(21) Anmeldenummer : **90100871.4**

(22) Anmeldetag : **17.01.90**

(54) **Verfahren zur Bestimmung von HIV-Antikörpern.**

(30) Priorität : **23.01.89 DE 3901857**

(43) Veröffentlichungstag der Anmeldung :
**01.08.90 Patentblatt 90/31**

(45) Bekanntmachung des Hinweises auf die
Patenterteilung :
**29.03.95 Patentblatt 95/13**

(84) Benannte Vertragsstaaten :
**AT BE CH DE DK ES FR GB GR IT LI LU NL SE**

(56) Entgegenhaltungen :
**EP-A- 0 326 490
EP-A- 0 344 578
WO-A- 88/08005
FR-A- 2 543 972**

(73) Patentinhaber : **BOEHRINGER MANNHEIM
GMBH
D-68298 Mannheim (DE)**

(72) Erfinder : **Klein, Christian, Dr.
Blütenstrasse 16
D-8120 Weilheim (DE)**
Erfinder : **Bayer, Hubert, Dr.
Am Ölschlag 2
D-8120 Weilheim (DE)**

EP 0 379 949 B1

**Beschreibung**

Die Erfindung betriffl ein Verfahren zur Bestimmung von Antikörpern gegen HIV und ein hierfür geeignetes Reagenz.

Die Bestimmung von HIV-Antikörpern ist eine wichtige diagnostische Aufgabe.

Aus WO 88/08005 und Science 237 (1987), 1346 - 1349, sind Peptidsequenzen bekannt, mit denen Antikörper gegen HIV-2 über Immunoassays bestimmt werden können. Bei den dort beschriebenen Bestimmungsverfahren müssen diese synthetischen Peptide jedoch in hohen Konzentrationen eingesetzt werden und es ergeben sich bei hohen Leerwerten nur geringe Signale.

Aus dem Abstract "DEVELOPMENT OF HIV-1 AND HIV-2 SPECIFIC AND SELECTIVE ENZYME LINKED IMMUNOSORBENT ASSAY BASED ON TRI-PALMITOYL-S-GLYCERYL-CYSTEINYL-PEPTIDES" vom 20th EUROPEAN PEPTIDE SYMPOSIUM, September 4 - 9, 1988 in Tübingen/FRG ist bekannt, daß bei Cyclisierung eines HIV-2-Peptids über zwei darin enthaltenen Cysteinreste die Empfindlichkeit verbessert werden kann. Das dort beschriebene $Pam_3Cys$-Peptid-Konjugat ist jedoch auch nur bedingt für einen empfindlichen HIV-2-Test geeignet. Zur Immobilisierung sind hier ebenfalls große Mengen des Konjugats erforderlich, und es ergeben sich bei zwar verbesserter Empfindlichkeit nach wie vor sehr hohe Leerwerte.

Cyclische HIV 2 und HIV-1-Peptide sind auch aus der EP-A-0 326 490, veröffentlicht am 02.08.1989, bekannt. Auch diese Peptide müssen für einen immunologischen Test in großer Menge eingesetzt werden und ergeben sehr hohe Leerwerte.

Aufgabe der vorliegenden Erfindung ist es deshalb, ein Verfahren zur Bestimmung von HIV-Antikörpern bereitzustellen, mit dem diese Nachteile beseitigt werden können.

Gegenstand der Erfindung ist deshalb ein Verfahren zur Bestimmung von HIV-Antikörpern nach dem Prinzip des heterogenen Immunoassays, welches dadurch gekennzeichnet ist, daß die Probe gleichzeitig oder sequenziell in einem Reaktionsgefäß, an dessen Innenoberfläche Streptavidin in einer solchen Menge gebunden ist, daß pro ml Reaktionsvolumen 0,1 - 2,5 µg vorhanden sind,

a) mit mindestens einem biotinylierten Polypeptid, welches aus maximal 50 Aminosäureresten besteht und die Aminosäuresequenz

$$\text{Cys-Ala-Phe-Arg-Gln-Val-Cys}$$

enthält, wobei jeweils die beiden Cysteine der Sequenz zu intramolekularen Cystinbrücken cyclisiert sind
b) sowie mit einem markierten Rezeptor, der gegen HIV-Antikörper gerichtet ist,

inkubiert, flüssige und feste Phase getrennt und in einer der beiden Phasen die Markierung als Maß für den HIV-Antikörper bestimmt wird.

Vorzugsweise wird zur Bestimmung von HIV-2-Antikörpern ein biotinyliertes Polypeptid verwendet, welches die Aminosäuresequenz Cys-Ala-Phe-Arg-Gln-Val-Cys enthält.

Es zeigt sich überraschenderweise, daß bei Verwendung der Streptavidin-Festphase, in Kombination mit biotinylierten monomeren cyclischen HIV-Peptiden, bei geringsten Einsatzmengen der Peptide hohe Empfindlichkeiten bei geringen Leerwertsignalen beobachtet werden.

Die Streptavidin-Festphase ist in der EP-A-0 344 578, veröffentlicht am 06.12.1989, beschrieben. Zur Bestimmung einer immunologisch nachweisbaren Substanz nach dem Prinzip des heterogenen Immunoassays wird eine Festphase benutzt, an deren Innenoberfläche Streptavidin oder Avidin in einer solchen Menge gebunden ist, daß pro ml Reagenzvolumen 0,1 - 2,5 µg vorhanden sind. Geeignete Polypeptidsequenzen sind in WO 88/08005, EP-A-0 292 454, EP-A-0 283 327 und EP-A-0 326 490, veröffentlicht cm 02.08.89, beschrieben. Die dort angeführten Sequenzen können nach Biotinylierung, Cyclisierung und Isolierung der biotinylierten monomeren cyclischen Peptide erfindungsgemäß verwendet werden.

Besonders geeignete biotinylierte Peptide sind Verbindungen der Formeln:

$$\text{X-A-Asn-Ser-Trp-Gly-Cys-Ala-Phe-Arg-Gln-Val-Cys-His-Thr-Thr} \quad \text{oder}$$

$$\text{Asn-Ser-Trp-Gly-Cys-Ala-Phe-Arg-Gln-Val-Cys-His-B-Y}$$

wobei

X   ein Biotinylaminocapronsäure-, Biotinylaminobuttersäure-, Biotinyl-, N-epsilon-Biotinyllysin-, N-epsilon(Biotinylaminocaprol)lysin-, N-delta-Biotinyl-ornithin- oder N-delta(Biotinylaminocaproyl)ornithinrest;

Y   ein N-epsilon-Biotinyllysin-, N-epsilon(Biotinylaminocaproyl)lysin-, N-delta-Biotinylornithin- oder N-delta(Biotinylaminocaproyl)ornithinrest;

A     eine Bindung oder eine Peptidsequenz wie Gln-Ala-Arg-Leu, Ala-Arg-Leu, Arg-Leu oder Leu und

B     eine Bindung oder eine Peptidsequenz wie Thr-Thr-Val-Pro-Trp, Thr-Thr-Val-Pro, Thr-Thr-Val, Thr-Thr oder Thr

sein kann.

Die Herstellung der biotinylierten Peptide kann beispielsweise durch Synthese der Peptide nach Merrifield JACS 85 (1964), 2146 erfolgen. Die Biotinylierung kann beispielsweise nach PNAS 80 (1983), 4045 erfolgen. Die Peptide werden vor oder nach Biotinylierung durch Oxidation cyclisiert und das Monomer durch Chromatographie, vorzugsweise HPLC-Chromatographie, isoliert.

Die Herstellung der Peptide kann erfolgen, indem man zuerst die entsprechenden Peptidsequenzen synthetisiert, anschließend die Peptide durch Oxidation der Cystein-SH-Gruppen zu Cystin-Peptiden cyclisiert und danach mit einem gängigen Biotinylierungsmittel, z. B. Biotinylaminocapronsäure-N-hydroxysuccinimidester, biotinyliert, oder indem man die Peptide zunächst biotinyliert und anschließend cyclisiert.

Die Peptide können z. B. durch Festphasensynthese hergestellt werden z.B. mit temporären tert. Butyloxycarbonyl-Schutz der alpha-Aminosäuren (Merrifield, JACS 85 (1964), 2146) oder mit temporärem Fluorenylmethoxycarbonyl-Schutz ("Fmoc") (Meienhofer, Int. J. Pept. Prot. Res 11(1978), 246).

Eine weitere Möglichkeit der Herstellung der erfindungsgemäßen Peptide ist die Einführung des Biotinrestes während der Festphasensynthese, z. B. indem man N-alpha-Fmoc-N-epsilon(biotinylaminocaproyl)lysin, N-alpha-Fmoc-N-epsilon-Biotinyllysin oder bei Biotinylierung der N-terminalen Aminosäure Biotin-N-hydroxysuccinimidester oder Biotinylaminocapronsäure-N-hydroxysuccinimidester einsetzt.

Eine weitere besonders bevorzugte Herstellungsmethode ist die Cyclisierung der Peptide nach der Festphasensynthese noch am polymeren Träger. Dazu setzt man an der SH-Gruppe durch den Tritylrest geschütztes Cystein ein und behandelt die festphasengebundenen Peptide mit Jod in einem Lösungsmittelgemisch, das eine perfluorierte Verbindung enthält, bevorzugt Dichlormethan/Hexafluorisopropanol. Bei dieser Methode erhält man praktisch ausschließlich cyclisierte monomere Peptide und praktisch keine Polymere.

Vorzugsweise werden die biotinylierten monomeren Peptide in einer Konzentration von 0,005 - 0,4 µg/ml, besonders bevorzugt in einer Konzentration von 0,02 - 0,3 µg/ml, eingesetzt. Die Länge der Peptide ist maximal 50 Aminosäuren, bevorzugt maximal 35 und besonders bevorzugt maximal 20 Aminosäuren.

Als markierter Rezeptor wird bevorzugt ein Antikörper verwendet, der gegen den Fc$\gamma$-Teil von humanem IgG gerichtet ist. Dieser Antikörper kann sowohl monoklonal als auch polyklonal sein. Bevorzugt wird ein Antikörper verwendet, der gegen den Fc$\gamma$-Teil von humanem IgG gerichtet ist.

Als Markierung können die dem Fachmann geläufigen Markierungen, wie beispielsweise Enzyme (z. B. Peroxidase, alkalische Phosphatase), Radionukleotide, fluorogene oder chromogene Substrate, Cofaktoren, koloidales Gold oder magnetische Partikel verwendet werden.

Die Durchführung des erfindungsgemäßen Verfahrens erfolgt in der Weise, daß die Probe und biotinyliertes Antigen zu einer Streptavidin-Festphase zugegeben werden, inkubiert und gewaschen wird. Anschließend wird markierter Antikörper gegen HIV-1- und/oder HIV-2-Antikörper zugegeben, inkubiert, gewaschen und die Markierung in der Festphase oder der abgetrennten flüssigen Phase bestimmt.

Eine Variante des Bestimmungsverfahrens besteht darin, daß als markierte Rezeptoren markierte Polypeptide, welche aus maximal 50 Aminosäureresten bestehen und die Aminosäuresequenz Cys-Ala-Phe-Arg-Gln-Val-Cys, vorzugsweise Cys-Ala-Phe-Arg-Gln-Val-Cys enthalten, wobei jeweils die beiden Cysteine der Aminosäuresequenzen zu Cystinbrücken cyclisiert sind und das Polypeptid monomer ist, verwendet werden.

Bevorzugt werden verwendet Peptide der Formeln:

X-A-Asn-Ser-Trp-Gly-Cys-Ala-Phe-Arg-Gln-Val-Cys-His-Thr-Thr     oder

Asn-Ser-Trp-Gly-Cys-Ala-Phe-Arg-Gln-Val-Cys-His-B-Y

wobei

X     ein Biotinylaminocapronsäure-, Biotinylaminobuttersäure-, Biotinyl-, N-epsilon-Biotinyllysin-, N-epsilon(Biotinylaminocaprol)lysin-, N-delta-Biotinyl-ornithin- oder N-delta(Biotinylaminocaproyl)ornithinrest;

Y     ein N-epsilon-Biotinyllysin-, N-epsilon(Biotinylaminocaproyl)lysin-, N-delta-Biotinylornithin- oder N-delta(Biotinylaminocaproyl)ornithinrest;

A     eine Bindung oder eine Peptidsequenz wie Gln-Ala-Arg-Leu, Ala-Arg-Leu, Arg-Leu oder Leu und

B     eine Bindung oder eine Peptidsequenz wie Thr-Thr-Val-Pro-Trp, Thr-Thr-Val-Pro, Thr-Thr-Val, Thr-Thr oder Thr

sein kann.

Die nachfolgenden Beispiele erläutern die Erfindung weiter:

**Beispiel 1**

Synthese von Biotinylaminocaproyl-Asn-Ser-Trp-Gly-Cys-Ala-Phe-Arg-Gln-Val-Cys-His-Thr-Thr, monomer, cyclisch.

Analog Meienhofer (s.o.) wird das Peptid AsnSer(tBu)TrpGlyCys(Trt)AlaPheArg(Mtr)GlnValCys(Trt)His(Trt)Thr(tBu) an der Festphase synthetisiert. Danach wird das Peptid-Harz in dem 100fachen Volumen mit jodgestättigtem Dichlormethan/ Hexafluorisopropanol (3:1) 2 Std. geschüttelt und je dreimal mit Dichlormethan, Dimethylformamid und Isopropanol gewaschen. Danach wird das Peptid wie bei Meienhofer beschrieben vom Harz abgespalten, mit Biotinylaminocapronsäure-N-hydroxysuccinimidester biotinyliert und über präparative HPLC gereinigt.

FAB-MS (positiv): $MH^+$ = 1947

Nach Spaltung mit Dithiothreitol erhöht sich die Retentionszeit im HPLC (Reversed Phase RP 18, Gradient 0.1 % Trifluoressigsäure in Wasser gegen 0.1 % Trifluoressigsäure in Wasser Acetonitril 40:60).

**Beispiel 2**

Verfahren zur Bestimmung von HIV-2-Antikörpern

Reagenz 1

| | |
|---|---|
| 0 - 0,3 µl | Biotinylaminocaproyl-Asn-Ser-Trp-Gly-Cys-Ala-Phe-Arg-Gln-Val-Cys-His-Thr-Thr    (Peptid 1, Herstellung nach Beispiel 1) |
| 40 mmol/l | Phosphatpuffer pH 7.0 |
| 0,9 Gew.-% | Kochsalz |
| 10 Vol.-% | Rinderserumalbumin |

Reagenz 2

| | |
|---|---|
| 20 mU/ml | eines Konjugates aus Peroxidase und einem polyklonalen Schafantikörper gegen humanes Immunglobulin |
| 40 mmol/l | Phosphatpuffer pH 7,0 |
| 0,05 Gew.-% | Tween® 20 |
| 0,2 % | Rinderserumalbumin |
| 0,2 % | Rinder-IgG |

In einem mit Streptavidin beschichteten Polystyrolröhrchen (hergestellt nach Beispiel 1 von DE 38 17 716) werden 1 ml Reagenz 1 und 10 µl Probe eine Stunde bei Raumtemperatur inkubiert. Anschließend wird dreimal mit Leitungswasser gewaschen, mit 1 ml Reagenz 2 für eine Stunde bei Raumtemperatur inkubiert und wieder dreimal mit Leitungswasser gewaschen. Zur Nachweisreaktion werden 1 ml ABTS® (2,2'-Azino-di-[3-ethyl-benzthiazolin-sulfonsäure(6)]-diammoniumsalz) in 100 mmol/l Phosphatcitratpuffer pH 4,4 mit 3,2 mmol/l Natriumperborat zugegeben. Nach 60 Minuten wird die Extinktion bei 422 nm photometrisch gemessen. Für die unterschiedlichen Konzentrationen von Peptid 1 werden folgende Ergebnisse erhalten:

| Konzentration Peptid 1 | Extinktion bei 422 nm |
|---|---|
| 0 µg/ml | 120 mE |
| 0,002 µg/ml | 240 mE |
| 0,005 µg/ml | 690 mE |
| 0,01 µg/ml | 1.150 mE |
| 0,04 µg/ml | 1.570 mE |
| 0,07 µg/ml | 1.570 mE |
| 0,1 µg/ml | 1.570 mE |
| 0,3 µg/ml | 1.330 mE |

**Patentansprüche**

1. Verfahren zur Bestimmung von HIV-Antikörpern nach dem Prinzip des heterogenen Immunoassays, dadurch gekennzeichnet, daß die Probe gleichzeitig oder sequenziell in einem Reaktionsgefäß an dessen Innenoberfläche Streptavidin in einer solchen Menge gebunden ist, daß pro ml Reaktionsvolumen 0,1 - 2,5 µg vorhanden sind,
   a) mit mindestens einem biotinylierten Polypeptid, welches aus maximal 50 Aminosäureresten besteht und die Aminosäuresequenz Cys-Ala-Phe-Arg-Gln-Val-Cys enthält, wobei jeweils die beiden Cysteine dieser Aminosäuresequenz zu Cystinbrücken cyclisiert sind und das Polypeptid monomer ist
   b) sowie mit einem markierten Rezeptor, der gegen den HIV-Antikörper gerichtet ist,
   inkubiert, flüssige und feste Phase getrennt und in einer der beiden Phasen die Markierung als Maß für HIV-Antikörper bestimmt wird.

2. Verfahren nach Anspruch 1, dadurch gekennzeichnet, daß als markierter Rezeptor ein Antikörper verwendet wird, der gegen den Fcγ-Teil von humanen Immunglobulin gerichtet ist.

3. Verfahren nach Anspruch 1 oder 2, dadurch gekennzeichnet, daß als biotinyliertes Polypeptid ein Peptid der Formeln:

X-A-Asn-Ser-Trp-Gly-Cys-Ala-Phe-Arg-Gln-Val-Cys-His-Thr-Thr   oder

Asn-Ser-Trp-Gly-Cys-Ala-Phe-Arg-Gln-Val-Cys-His-B-Y

verwendet wird, wobei

X    ein Biotinylaminocapronsäure-, Biotinylaminobuttersäure-, Biotinyl-, N-epsilon-Biotinyllysin-, N-epsilon(Biotinylaminocaproyl)lysin-, N-delta-Biotinylornithin- oder N-delta-(Biotinylaminocaproyl)ornithinrest;

Y    ein N-epsilon-Biotinyllysin-, N-epsilon(Biotinylaminocaproyl)lysin-, N-delta-Biotinylornithin- oder N-delta(Biotinylaminocaproyl)ornithinrest;

A    eine Bindung oder eine Peptidsequenz wie Gln-Ala-Arg-Leu, Ala-Arg-Leu, Arg-Leu oder Leu und

B    eine Bindung oder eine Peptidsequenz wie Thr-Thr-Val-Pro-Trp, Thr-Thr-Val-Pro, Thr-Thr-Val, Thr-Thr oder Thr

sein kann.

4. Verfahren nach Anspruch 1 oder 3, dadurch gekennzeichnet, daß als markierter Rezeptor ein markiertes Polypeptid verwendet wird, welches aus maximal 50 Aminosäureresten besteht und die Aminosäuresequenz Cys-Ala-Phe-Arg-Gln-Val-Cys enthält, wobei jeweils die beiden Cysteine dieser Aminosäuresequenz zu Cystinbrücken cyclisiert sind und das Polypeptid monomer ist.

5. Biotinyliertes Polypeptid der Formeln:

$$\text{X-A-Asn-Ser-Trp-Gly-Cys-Ala-Phe-Arg-Gln-Val-Cys-His-Thr-Thr} \quad \text{oder}$$

$$\text{Asn-Ser-Trp-Gly-Cys-Ala-Phe-Arg-Gln-Val-Cys-His-B-Y}$$

wobei jeweils die beiden Cysteine dieser Polypeptide zu Cystinbrücken cyclisiert sind, und wobei

X   ein Biotinylaminocapronsäure-, Biotinylaminobuttersäure-, Biotinyl-, N-epsilon-Biotinyllysin-, N-epsilon(Biotinylaminocaproyl)lysin-, N-delta-Biotinylornithin- oder N-delta-(Biotinylaminocaproyl)ornithinrest;

Y   ein N-epsilon-Biotinyllysin-, N-epsilon(Biotinylaminocaproyl)lysin-, N-delta-Biotinylornithin- oder N-delta(Biotinylaminocaproyl)ornithinrest;

A   eine Bindung oder eine Peptidsequenz wie Gln-Ala-Arg-Leu, Ala-Arg-Leu, Arg-Leu oder Leu und

B   eine Bindung oder eine Peptidsequenz wie Thr-Thr-Val-Pro-Trp, Thr-Thr-Val-Pro, Thr-Thr-Val, Thr-Thr oder Thr

sein kann.

## Claims

1. Process for the determination of HIV antibodies according to the principle of the heterogenous immunoassay, characterised in that the sample is incubated simultaneously or sequentially in a reaction vessel on the inner surface of which is bound streptavidin in such an amount that, per ml of reaction volume, 0.1 - 2.5 µg are present,

   a) with at least one biotinylated polypeptide which consists of maximum 50 amino acid residues and the amino acid sequence contains Cys-Ala-Phe-Arg-Gln-Val-Cys, whereby in each case the two cysteines of this amino acid sequence are cyclised to cystine bridges and the polypeptide is monomeric

   b) as well as with a labelled receptor which is directed against the HIV antibodies,

   liquid and solid phase are separated and the labelling is determined in one of the two phases as measure for HIV antibodies.

2. Process according to claim 1, characterised in that, as labelled receptor, an antibody is used which is directed against the Fc$\gamma$ part of human immuno-globulin.

3. Process according to claim 1 or 2, characterised in that, as biotinylated polypeptide, a peptide of the formula:

$$\texttt{X-A-Asn-Ser-Trp-Gly-Cys-Ala-Phe-Arg-Gln-Val-Cys-His-}$$

$$\texttt{Thr-Thr or}$$

$$\texttt{Asn-Ser-Trp-Gly-Cys-Ala-Phe-Arg-Gln-Val-Cys-His-B-Y}$$

is used, whereby

X can be a biotinylaminocaproic acid, biotinylaminobutyric acid, biotinyl, N-epsilon-biotinyllysine, N-epsilon-(biotinylaminocaproyl)-lysine, N-delta-biotinylornithine or N-delta-(biotinylaminocaproyl)-ornithine residue;

Y an N-epsilon-biotinyllysine, N-epsilon-(biotinylaminocaproyl)-lysine, N-delta-biotinylornithine or N-delta-(biotinylaminocaproyl)-ornithine residue;

A a bond or a peptide sequence, such as Gn-Ala-Arg-Leu, Ala-Arg-Leu, Arg-Leu or Leu and

B a bond or a peptide sequence, such as Thr-Thr-ValPro-Trp, Thr-Thr-Val-Pro, Thr-Thr-Val, Thr-Thr or Thr.

4. Process according to claim 1 or 3, characterised in that, as labelled receptor, a labelled polypeptide is used which consists of maximum 50 amino acid residues and contains the amino acid sequence Cys-Ala-Phe-Arg-Gln-Val-Cys, whereby in each case the two cysteines of this amino acid sequence are cyclised to cystine bridges and the polypeptide is monomeric.

5. Biotinylated polypeptide of the formulae:

```
X-A-Asn-Ser-Trp-Gly-Cys-Ala-Phe-Arg-Gln-Val-Cys-His-
Thr-Thr  or
Asn-Ser-Trp-Gly-Cys-Ala-Phe-Arg-Gln-Val-Cys-His-B-Y
```

whereby in each case the two cysteines of this polypeptide are cyclised to cystine bridges and whereby X can be a biotinylaminocaproic acid, biotinylaminobutyric acid, biotinyl, N-epsilon-biotinyllysine, N-epsilon-(biotinylaminocaproyl)-lysine, N-delta-biotinylornithine or N-delta-(biotinylaminocaproyl)-ornithine residue;
Y an N-epsilon-biotinyllysine, N-epsilon-(biotinylaminocaproyl)-lysine, N-delta-biotinylornithine or N-delta-(biotinylaminocaproyl)-ornithine residue, A a bond or a peptide sequence, such as Gln-Ala-Arg-Leu, Ala-Arg-Leu, Arg-Leu or Leu and
B a bond or a peptide sequence, such as Thr-Thr-ValPro-Trp, Thr-Thr-Val-Pro, Thr-Thr-Val, Thr-The or Thr.

## Revendications

1. Procédé de détermination d'anticorps anti-HIV selon le principe des dosages immunologiques hétérogènes, caractérisé en ce que l'on incube les échantillons, de façon simultanée ou séquentielle, dans un récipient réactionnel à la surface interne duquel est fixée de la streptavidine, en une quantité telle qu'il y en ait 0,1-2,5 μg par ml de volume réactionnel,
    a) avec au moins un polypeptide biotinylé, qui est constitué d'au plus 50 résidus d'acides aminés et comprend la séquence d'acides aminés Cys-Ala-Phe-Arg-Gln-Val-Cys, les deux cystéines de cette séquence d'acides aminés étant cyclisées chacune en ponts cystine et le polypeptide étant un monomère,
    b) ainsi qu'avec un récepteur marqué, qui est dirigé contre l'anticorps anti-HIV,
on sépare les phases liquides et solides et dans l'une des deux phases, on détermine le marqueur, qui constitue une mesure de l'anticorps anti-HIV.

2. Procédé selon la revendication 1, caractérisé en ce que comme récepteur marqué, on utilise un anticorps qui est dirigé contre la partie Fcγ de l'immunoglobuline humaine.

3. Procédé selon la revendication 1 ou 2, caractérisé en ce que comme polypeptide biotinylé, on utilise un peptide de formule :

```
X-A-Asn-Ser-Trp-Gly-Cys-Ala-Phe-Arg-Gln-Val-Cys-His-Thr-Thr          ou
Asn-Ser-Trp-Gly-Cys-Ala-Phe-Arg-Gln-Val-Cys-His-B-Y
```

dans laquelle
X      peut représenter un reste d'acide biotinylaminocaproïque, d'acide biotinylaminobutyrique, de biotinyle, de N-epsilon-biotinyllisyne, de N-epsilon(biotinylaminocaproyl)lysine, de N-delta-biotinylornithine ou de N-delta-(biotinylaminocaproyl)ornithine ;
Y      peut représenter un reste de N-epsilon-biotinyllisyne, de N-epsilon-(biotinylaminocaproyl)lysine, de N-delta-biotinylornithine ou de N-delta-(biotinylaminocaproyl)ornithine;
A      peut représenter une liaison ou une séquence peptidique telle que Gln-Ala-Arg-Leu, Ala-Arg-Leu, Arg-Leu ou Leu et

B       peut représenter une liaison ou une séquence peptidique telle que Thr-Thr-Val-Pro-Trp, Thr-Thr-Val-Pro, Thr-Thr-Va, Thr-Thr ou Thr.

4.   Procédé selon la revendication 1 ou 3, caractérisé en ce que comme récepteur marqué, on utilise un polypeptide marqué qui est constitué d'au plus 50 résidus d'acides aminés et comprend la séquence d'acides aminés Cys-Ala-Phe-Arg-Gln-Val-Cys, les deux cystéines de cette séquence d'acides aminés étant cyclisées chacune en ponts cystine et le polypeptide étant un monomère.

5.   Polypeptide biotinylé de formule :

X-A-Asn-Ser-Trp-Gly-Cys-Ala-Phe-Arg-Gln-Val-Cys-His-Thr-Thr          ou

Asn-Ser-Trp-Gly-Cys-Ala-Phe-Arg-Gln-Val-Cys-His-B-Y

dans laquelle les deux cystéines de ce polypeptide sont cyclisées chacune en ponts cystine, et
dans laquelle

X       peut représenter un reste d'acide biotinylaminocaproïque, d'acide biotinylaminobutyrique, de biotinyle, de N-epsilon-biotinyllisyne, de N-epsilon(biotinylaminocaproyl)lysine, de N-delta-biotinylornithine ou de N-delta-(biotinylaminocaproyl)ornithine ;

Y       peut représenter un reste de N-epsilon-biotinyllisyne, de N-epsilon-(biotinylaminocaproyl)lysine, de N-delta-biotinylornithine ou de N-delta-(biotinylaminocaproyl)ornithine ;

A       peut représenter une liaison ou une séquence peptidique telle que Gln-Ala-Arg-Leu, Ala-Arg-Leu, Arg-Leu ou Leu et

B       peut représenter une liaison ou une séquence peptidique telle que Thr-Thr-Val-Pro-Trp, Thr-Thr-Val-Pro, Thr-Thr-Val, Thr-Thr ou Thr.